# EUROPEAN PATENT APPLICATION

(11) **EP 2 143 422 A1**
(43) Date of publication of application: **13.01.2010**
(21) Application number: 08425479.6
(22) Date of filing: 11.07.2008
(51) Int. Cl.: A61K 9/06, A61K 9/08, A61K 9/107, A61K 31/4375, A61K 31/545, A61K 31/573, A61K 31/7048, A61K 45/06, A61P 27/02, A61P 31/04

(54) **Ophthalmic compositions containing gemifloxacin for the treatment of ocular infections**

(71) Applicant: S.I.F.I - SOCIETÀ INDUSTRIA FARMACEUTICA ITALIANA - S.P.A., Frazione Lavinaio 95020 Aci S. Antonio (IT)
(72) Inventor: Blanco, Anna Rita, SANTA MARIA DEGLI AMMALATI, 95024 ACIREALE (IT); Sudano Roccaro, Andrea, 95024 ACIREALE (IT); Civiale, Claudine, 95022 ACICATENA (IT)
(74) Representative: Gervasi, Gemma

(57) **Abstract**

Pharmaceutical compositions suitable for treating and preventing ocular infections, characterized by the use of gemifloxacin as the active principle, possibly in combination with other antibiotics and with other steroidal or non-steroidal antiinflammatory drugs.

## Description

### FIELD OF THE INVENTION

The present invention relates to pharmaceutical compositions for the treatment of ocular infections. In particular the invention relates to formulations containing gemifloxacin, a new fourth generation fluoroquinolone, or derivatives thereof, optionally in combination with other active principles commonly used for the prevention or treatment of ocular infections (infections of the cornea, conjunctiva, ocular adnexae, uvea and choroid) such as: other antibiotics able to broaden its spectrum of action (penicillins, cephalosporins, macrolides, aminoglycosides), steroidal and non-steroidal anti-inflammatories.

The compositions of the invention can also be used in the case of refractive, cataract or vitrectomy surgeries with the aim of preventing the onset of endophthalmitis, a rare but serious complication associated with ophthalmic surgery.

Further possible applications of the present invention are: a) in cataract operations - where the crystalline lens is replaced by an intraocular lens (IOL) - the preparation of medicated IOLs impregnated with a composition containing gemifloxacin or its derivatives, to be used as a drug delivery system for the prophylaxis of endophthalmitis; b) the preparation of other delivery systems, such as corneal lenses impregnated with a gemifloxacin-containing composition to increase antibiotic penetration into the anterior chamber.

### STATE OF THE ART

Ophthalmic infections can have serious consequences for sight if not quickly and adequately treated. Antibiotics with a broad spectrum of action are therefore often used, depending on the clinical history of the patient and the pathogens most commonly involved in the development of infections of the ocular region. Ophthalmic infections of the external regions of the eye (blepharitis, conjunctivitis, keratitis) are generally treated with topical antibiotic-based compositions (as collyriums or ophthalmic ointments).

Compositions containing antibiotics can also be administered by subconjunctival injection or injection into the anterior chamber: these techniques are used in particular to alter pharmacokinetics and to achieve a slow-release transfer (subconjunctival injection) or to increase the concentration of active principle able to reach the site of action (injection into the anterior chamber) in the case of non-surface infections, e.g. for treating endophthalmitis.

Moreover, for many years bacterial keratitis was treated using cephalosporins or aminoglycosides as first choice drugs in "fortified" formulations, i.e. with high active principle concentrations, but this type of treatment was linked to high local toxicity and led to the onset of strains resistant to these antibiotic classes and to cross-resistance with other antibiotics.

In this respect it is known that microorganisms exposed to the action of antibacterial agents tend, having identified the action mechanism by which these eradicate them (bactericides) or prevent their proliferative capacity (bacteriostatics), to counteract this action by effecting genetic mutations allowing subsequent generations to resist the effect of these active principles. This process is known as "resistance".

The frequent and often indiscriminate use of antibiotics has therefore led to the onset of strains resistant to the most commonly used antibiotics and the need to always provide new active principles which can be effective on both Gram+ and Gram- bacteria.

In particular, for ophthalmic infections, it is important to use new active principles formulated in compositions which, when administered directly to the eye (for example at the surface or by local injection, or with medicated IOLs), enable effective active principle concentrations to be reached in the tissues of interest, with low systemic absorption and thus with a low incidence of unwanted systemic effects.

### SUMMARY OF THE INVENTION

The technical problem at the base of the present invention is hence to find a new effective antibiotic for ocular infections, based on the epidemiological data described in the literature regarding those bacterial strains most representative of ocular pathogens (Everett S.L. et al., Cornea 1995; 14:382-387; Starr C.E. et al., Invest Ophthalmol Vis Sci 2000; 41:S149; Ly C.N. et al., Clin Experiment Ophthalmol, 2006, 34 (1) 44-50).

In accordance with the present invention this problem is solved by the use of ophthalmic pharmaceutical compositions containing gemifloxacin.

The object of the invention is to provide ophthalmic pharmaceutical compositions useful for quickly and effectively resolving or preventing ocular infections, with good therapeutic safety due to low systemic absorption.

### BRIEF DESCRIPTION OF THE DRAWINGS

The characteristics and advantages of the present invention will become more evident from some embodiments given by way of non-limiting example, and illustrated by means of the diagrams shown in the accompanying figures wherein:

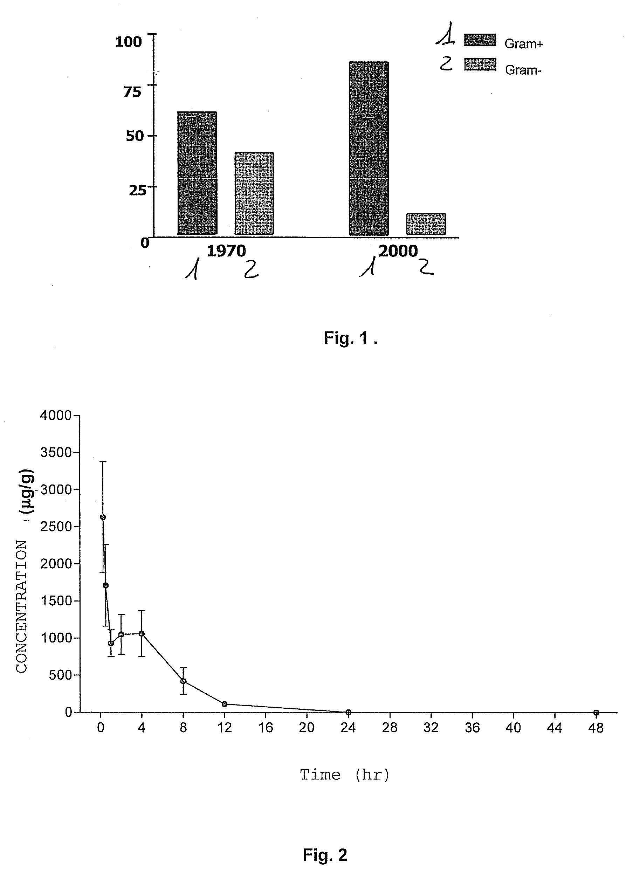

Figure 1 presents epidemiological data on the incidence of ocular bacterial infections;
Figure 2 is a graph of the distribution of gemifloxacin in the cornea after topical ocular single administration of a 0.3% aqueous solution;

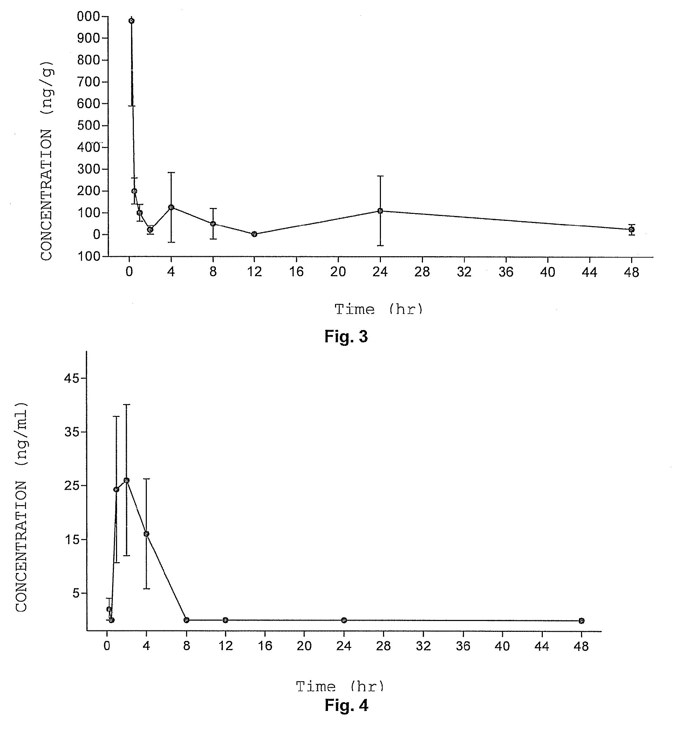

Figure 3 is a graph of the distribution of gemifloxacin in the conjunctiva after topical ocular single administration of a 0.3% aqueous solution;
Figure 4 is a graph of the distribution of gemifloxacin in the aqueous humour after topical ocular single administration of a 0.3% aqueous solution;

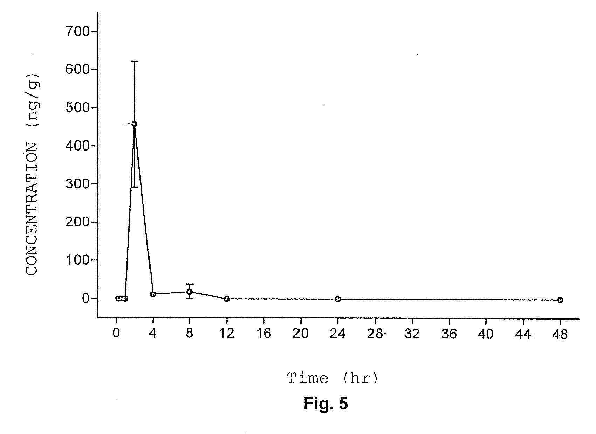

Figure 5 is a graph of the distribution of gemifloxacin in the iris-ciliary body after topical ocular single administration of a 0.3% aqueous solution;
Figure 6 is a graph of bacterial load reduction in the cornea after multi-administration of 0.3% gemifloxacin in aqueous solution compared to a 0.3% aqueous norfloxacin solution in a model of bacterial keratitis due to *St. aureus*;
Figure 7 is a graph of gemifloxacin accumulation in the cornea after multi-administration of 0.3% and 0.15% gemifloxacin in aqueous solution compared to a 0.3% aqueous norfloxacin solution in a model of bacterial keratitis due to St. aureus.
Figure 8 is a graph of gemifloxacin accumulation in the aqueous humour after multi-administration of 0.3% and 0.15% gemifloxacin in aqueous solution compared to a 0.3% aqueous norfloxacin solution in a model of bacterial keratitis due to *St. aureus.*
Figure 9 is a graph of bacterial load reduction in the cornea after multi-administration of 0.3% gemifloxacin in emulsion and in aqueous solution compared to a 0.3% aqueous ofloxacin solution in a model of bacterial keratitis due to *St. aureus.*
Figure 10 is a graph of gemifloxacin accumulation in the cornea after repeated administration of 0.3% gemifloxacin solution and emulsion compared with a 0.3% aqueous ofloxacin solution in a model of bacterial keratitis due to *St. aureus.*
Figure 11 is a graph of gemifloxacin accumulation in the aqueous humour after repeated administration of 0.3% gemifloxacin solution and emulsion compared with a 0.3% aqueous ofloxacin solution in a model of bacterial keratitis due to *St. aureus.*

### DETAILED DESCRIPTION OF THE INVENTION

Among the antibiotics used in the ophthalmic field, quinolones and especially fluoroquinolones are particularly important, even though this antibiotic class is correlated to a significant concentration-dependent retinal toxicity. Fluoroquinolones are synthetic antibiotics derived from nalidixic acid (a first generation quinolone). The different structural modifications brought to the quinolone nucleus with the various fluoroquinolone generations - 4 to date - have been established to overcome bacterial resistance having developed by exposure of bacterial strains to said active principles, and to broaden their spectrum of action.

Fluoroquinolones of the latest generation possess a dual mechanism of action: in fact, they interfere with both DNA gyrase (i.e. topoisomerase II) and topoisomerase IV, being enzymes involved in bacterial DNA replication. The characteristic mechanism of action of these antibacterial agents makes more difficult the arising of the resistance by the microorganisms, wherein 2 mutations must be expressed simultaneously for its effect to be hindered thereby.

Gemifloxacin is a fourth generation fluoroquinolone of formula 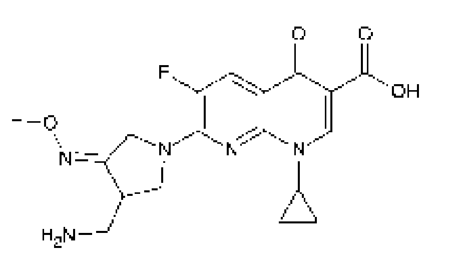 approved in Europe and the USA for the systemic treatment of respiratory infections. Like other fourth generation fluoroquinolones, gemifloxacin extends its spectrum of action to Gram+ bacteria while maintaining good activity against Gram- bacteria.

Following a number of studies and experiments carried out on the most widespread bacterial strains responsible for ocular infections, it has been surprisingly found that gemifloxacin, i.e. (R,S)-7-(3-aminomethyl-4-methoxy iminopyrrolidin-1yl)-1-cyclopropyl-6-fluoro-4-oxo-1,4-dihydro-1,8-naphthyridin-3-carboxylic acid, employed both as racemic mixture and separated *d* and /enantiomers, thereof and, additionally, both as acid and derivative (salts, esters, ethers) thereof, is found to be more effective *in vitro* against methicillin - susceptible and methicillin-resistant Gram+ and Gram- isolated ocular bacterial strains than third and fourth generation fluoroquinolones normally used in the ophthalmic field, such as ofloxacin, ciprofloxacin, levofloxacin and moxifloxacin.

**Table 1**

| MIC₅₀/MIC₉₀ (µg/ml) | | | | | |
|---|---|---|---|---|---|
| *Strain (No.)* | *Ofloxacin* | *Levofloxacin* | *Ciprofloxacin* | *Moxifloxacin* | *Gemifloxacin* |
| 27 *St. aureus* | 0.5/1.5 | 0.25/0.75 | 0.5/>32 | 0.064/0.38 | 0.032/0.064 |
| 29 *St. epidermidis* | 0.25/16 | 0.19/0.38 | 0.19/0.5 | 0.047/0.094 | 0.023/0.094 |
| 16 *St. coagulase negative* | 0.25/0.5 | 0.25/0.25 | 0.25/0.25 | 0.047/0.094 | 0.016/0.047 |
| 8 *Ps. aeruginosa* | 0.5/1 | 0.5/0.75 | 0.125/0.25 | 0.5/1 | 0.125/0.25 |
| 5 *Serratia marcescens* | 0.19/0.38 | 0.125/0.25 | 0.064/0.125 | 0.19/0.38 | 0.125/0.19 |

These data were confirmed in *in vivo* studies in a model of infective keratitis due to *St aureus,* and compared with norfloxacin and ofloxacin.

Effectiveness studies conducted *in vitro* on ocular isolates such as *St. aureus* (27 strains), *St. epidermidis* (29 strains), *Ps. aeruginosa* (8 strains), Coagulase Negative Staphylococci - CNS - (16 strains) and *S. marcescens* (5 strains) actually show that the MIC_{50/90} (minimum concentration to inhibit 50/90% of the bacterial population) of gemifloxacin is lower than the MIC_{50/90} of ciprofloxacin, ofloxacin, levofloxacin and moxifloxacin for the same strains, as can indeed be deduced from the data in Table 1. The present invention also concerns the optional use of gemifloxacin in combination with other possibly usable molecules for treating ocular infections of the cornea, conjunctiva, ocular adnexae, uvea and iris-choroid, such as penicillins, cephalosporins, macrolides aminoglycosides, steroidal and non-steroidal anti-inflammatories. Said compositions can also be used in case of refractive, cataract or vitrectomy surgery with the aim of preventing the onset of endophthalmitis, a rare but serious complication associated with ophthalmic surgery.

The ophthalmic compositions of the present invention comprise gemifloxacin in an amount of 0.01-1 %, preferably 0.05-0.5%, and even more preferably 0.15%-0.3%. Various ophthalmic pharmaceutical forms of gemifloxacin or its derivatives form part of the invention, including, as non-limiting examples: solutions, emulsions, ointments, medicated solid inserts, suspensions, gels, solutions obtained by means of cyclodextrins, liposomes, nanoparticles, micelles, for topical ocular application or for subconjunctival or intraocular injection.

The aforementioned compositions can also be used for preparing medicated IOLs for use in cataract surgery as lens replacements or medicated corneal lenses for treating infections of the anterior chamber.

The compositions of the invention can be supplied to the patient in single-dose or multi-dose packs, possibly containing excipients normally used in the preparation of products for ophthalmic use such as: buffers, antimicrobial preservatives, isotonizing agents, chelating agents.

The buffering agent can be chosen from those known in the ophthalmic field, such as buffers of phosphate, phosphate-citrate, Tris, NaOH, histidine, tricine, lysine, glycine, serine, in case adjusted to the correct pH with an acid component. The buffer is at a concentration such that a pH between 5 and 8 is obtained/maintained, being compatible with ocular tissue.

The isotonizing agent can be chosen from known ones, such as sodium chloride or citric acid, glycerol, sorbitol, mannitol, ethylene glycol, propylene glycol, dextrose, and is present within a concentration range, for example, of 0 to 1% w/v, rendering the composition isotonic with lacrimal fluid (270-310 mOsm/kg). The aforesaid buffering and isotonizing agents, although useful and preferred, are not imperative for the purposes of the present invention. Usable chelating agents include for example EDTA (ethylenediaminotetracetic acid).

The compositions of the invention formulated in multi-doses can also contain antimicrobial preservatives such as: parabens, quaternary ammonium salts, polyhexamethylene biguanidine (PHMB) and others from those usable in compositions for ophthalmic use.

The solvent used in the compositions is preferably water or an aqueous solution of one or more components compatible with topical ophthalmic use.

The following examples further illustrate the invention without limiting it.

### EXPERIMENTAL PART

### Example 1. Gemifloxacin in an aqueous solution for ophthalmic use

### 1.1 Composition

| **Gemifloxacin in solution** | **g % (w/w)** |
|---|---|
| Gemifloxacin | 0.1500 or 0.3000 |
| NaCl | 0.9000 |
| NaOH | q.b. to pH 5.8 |
| Water q.b. (as needed) | q.b. to 100 ml |

### Example 2. Gemifloxacin in an emulsion for ophthalmic use

### 2.1 Composition

| **Gemifloxacin in emulsion** | **g % (w/w)** |
|---|---|
| Gemifloxacin | 0.1500 or 0.3000 |
| Soya oil | 7.000 |
| Egg yolk phospholipids | 3.000 |
| Glycerol | 1.800 |
| TRIS base | 0.2420 |
| HCl | q.b. to pH 7.6 |
| Water q.b. (as needed) | q.b. to 100 ml |

### Example 3. Gemifloxacin in an ointment for ophthalmic use

### 3.1 Composition

| **Gemifloxacin in ophthalmic ointment** | **g % (w/w)** |
|---|---|
| Gemifloxacin | 0.1500 or 0.3000 |
| Liquid paraffin | 15.0000 |
| Anhydrous wool fat | 15% |
| Vaseline | q.b. to 100 g |

### Example 4. In vitro tests of antibacterial effectiveness

### Epsilometer-test (E-test)

### 4.1 Methodology

In order to assay the effectiveness of gemifloxacin against the most representative pathogenic strains in ophthalmic infections, its activity was verified *in vitro,* in terms of MIC₅₀ and MIC₉₀ with the E-test method described by V. Lorian in "Antibiotics in laboratory medicine" (IV ed. 1996, 23-28) and compared to moxifloxacin, ciprofloxacin, levofloxacin and ofloxacin against 85 ocular bacterial isolates subdivided as follows:
27 *St. aureus,* 29 *S. epidermidis,* 16 Coagulase Negative Staphylococci (CNS), 8 *Ps. aeruginosa* and 5 *Serratia marcescens.* The following ATCC bacterial strains were also included: *Staphylococcus aureus* 25923, *Staphylococcus aureus* 29213, *Staphylococcus aureus* 43300, *S. epidermidis* 35984, *Pseudomonas aeruginosa* 27853 and *Pseudomonas aeruginosa* 9027.

The staphylococci also included methicillin resistant strains (MRSA and MRSE).

### 4.2 Results

Table 1 shows the MIC values (Minimum Inhibitory Concentration) determined with the E-test, expressed, for each antibiotic and for each group of tested bacterial isolates, in terms of MIC₅₀ and MIC₉₀.

By comparing the MIC_{50/90} values for gemifloxacin with those obtained for fluoroquinolones of the same or a previous generation, it was found that gemifloxacin demonstrates greater effectiveness (a lower MIC_{50/90}) against all the tested strains. A lower MIC_{50/90} value indicates a better *in vivo* inhibition quotient (IQ=C.max in tissue/MIC₉₀) than the compared fluoroquinolones.

The inhibition quotient (IQ - Tissue concentration/MIC₉₀), which is a pharmacodynamics parameter expressing in numerical terms the actual ability of an antibiotic to exceed, in a precise region, the minimum concentration able to inhibit the development of a microorganism, must always be ≥1 to ensure an effective therapeutic action of the antibiotic (Regis P. et al., Ophthalmology, 2005, 112 (11) 1987e1-1987e6; Pierre YR et al., Drugs, 2001, 61 (2) 175-185); the IQs for gemifloxacin in the various tissues are always >1 even in single administration (Ref. *In vivo* pharmacokinetic tests).

### Example 5: In-vivo pharmacokinetic tests

### 5.1 Methodology

The penetration capacity of gemifloxacin was determined in the cornea, conjunctiva, aqueous humour and iris-ciliary body after single ophthalmic administration (30 µl) of the solution at 0.3% in both eyes of pigmented rabbits. The tissues were collected at the following times: 15 minutes, 30 minutes, 1, 2, 4, 8, 12, 24 and 48 hours. Quantitative analysis of the samples was carried out by a LC/MS/MS method after suitable extraction of the active principle from the tissue and expressed as µg/g or µg/ml of tissue.

### 5.2 Results

Cmax, tmax and AUC for each tissue are given in Table 2.

**Table 2**

| TISSUE | tₘₐₓ (min) | Cₘₐₓ (µg/g or µg/ml) | AUC (min^{*}µg/g) |
|---|---|---|---|
| cornea | 15 | 2.600 | 519.59 |
| conjunctiva | 15 | 0.980 | 199.10 |
| aqueous humour | 120 | 0.026 | 6.35 |
| iris-ciliary body | 120 | 0.460 | N.D. |

### 5.3 Conclusions

The data show that gemifloxacin, after single administration of the 0.3% aqueous solution in animals with healthy eyes, permeates poorly into the aqueous humour (Cmax=0.026 µg/ml), whereas it well permeates into the iris-ciliary body (Cmax=0.460 µg/g), the cornea (Cmax=2.600 µg/ml) and the conjunctiva (Cmax=0.980 µg/ml). In these three ocular regions IQ values - taking into account the MIC₉₀ for *St. aureus -* are much higher than 1: specifically, IQ_{iris-ciliary} body = 0.46/0.064 = 7.2, IQ_{cornea} = 2.63/0.064 = 41.1 and IQ_{conjunctiva} = 0.98/0.064 = 15.3.

### Example 6: In-vivo effectiveness tests and studies on accumulation in the cornea and aqueous humour

### 6.1 Methodology

The effectiveness and accumulation of gemifloxacin in the cornea and aqueous humour were determined *in vivo* after administration of the 0.3% gemifloxacin compositions (emulsion and aqueous solution) and compared to 0.3% norfloxacin and ofloxacin in aqueous solution, in New Zealand albino rabbits having been induced with bacterial keratitis (Survey of Ophthalmology, vol. 50, suppl. Nov. 2005, p.S32-S48) by intrastromal injection of an inoculum of an ocular *St. aureus* isolate (7786).

Male New Zealand albino rabbits were used (Harlan Italia) into which the keratitis had been induced - following anaesthesia with ketamine (37.5 mg/kg) and xylazine (10 mg/kg) by intramuscular means and novesine by topical means (2 drops per eye) - by intrasomal injection of a suspension of the ocular *St. aureus* isolate 7786 in PBS (Phosphate Buffered Solution). The injection was carried out using an insulin syringe with a 30-gauge needle in each cornea of each rabbit.

Two experiments were carried out.

In the first experiment the following treatments were compared: 0.15% aqueous gemifloxacin solution, 0.3% aqueous gemifloxacin solution, 0.3% aqueous norfloxacin solution and physiological solution (0.9% NaCl) as control. The treatment was undertaken every 2 hours over 3 consecutive treatment days (5 administrations/day). The tissues were collected an hour after the last treatment, with the end point being after one day and after three days of treatment.

In the second experiment the following treatment groups were compared: 0.3% aqueous gemifloxacin solution, 0.3% gemifloxacin emulsion, 0.3% aqueous ofloxacin solution and physiological solution (0.9% NaCl) as control. The treatment was carried out every hour for only one day of treatment (7 administrations/day). The tissues were collected from the animals in each group, killed one hour after the last treatment.

All the compositions used in the two experiments described were administered at 50 µl per treatment.

Immediately after the animals had been killed, the aqueous humours were withdrawn (with a hypodermic syringe) and the corneas collected.

The bacterial load of each sample was determined by counting CFUs (Colony Forming Units) on a filter and plate, to be reported as CFU/g of tissue.

The determination of each antibiotic used in the aforesaid studies in the cornea (after suitable homogenization of the tissue and extraction of the active principle) and aqueous humour was carried out by adapting the spectrofluorimetric method applied by Ocaña et al. for determining moxifloxacin in human serum and urine (Analyst, 2000 Dec; 125 (12) 2322:2325), with reference to a standard curve.

The fluorescence value obtained was interpolated using the relative standard curve and expressed as nanograms/ml or nanograms/g of tissue.

### 6.2 Results

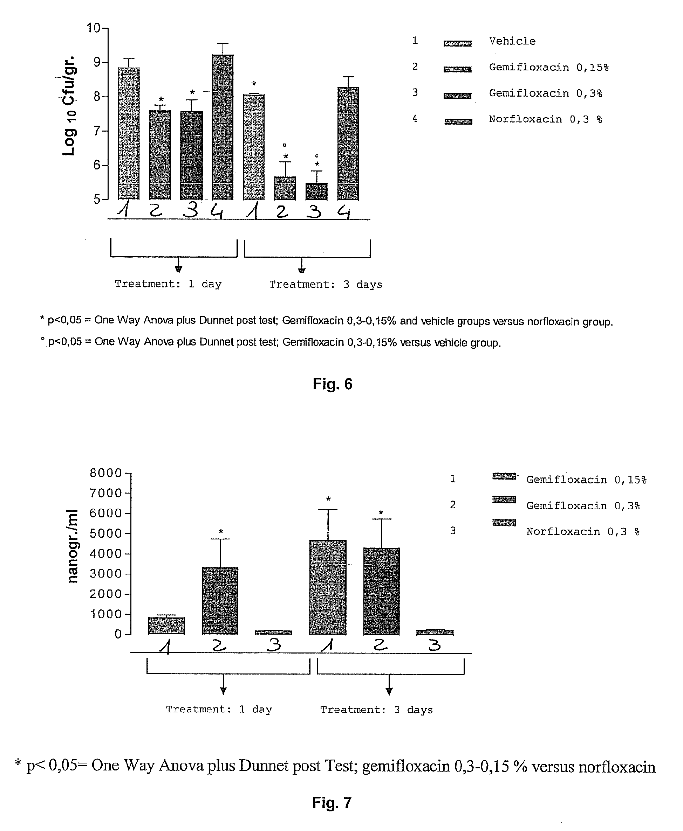

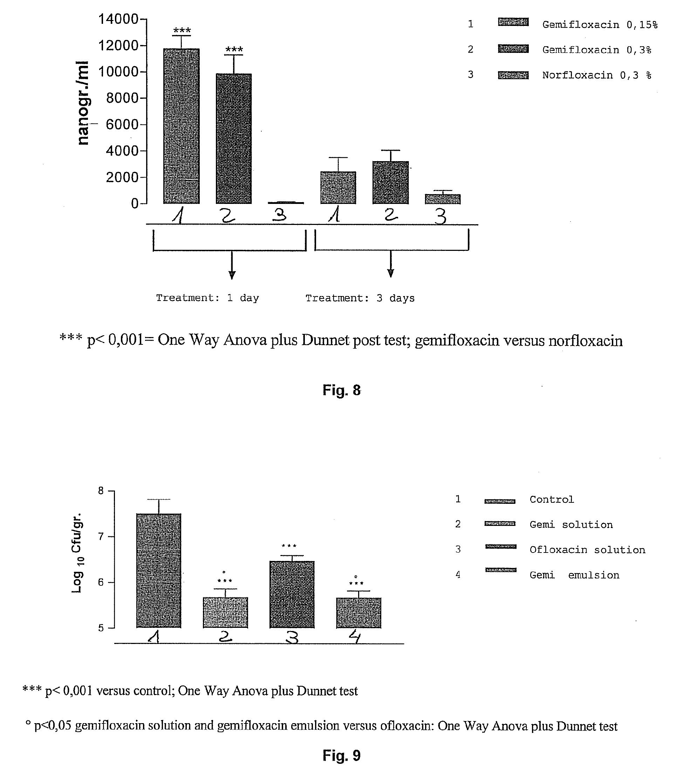

The experimental data relating to the first experiment (on which the One-way Anova and Dunnet Post Test statistical analyses were undertaken) were expressed as graphs in Figures 6, 7 and 8 and those relating to the second experiment, in Figures 9, 10, and 11.

### 6.2.1 Reduction of microbial load

The graph relating to the first experiment (Fig. 6) shows that in the model used, treatment with 0.3% norfloxacin solution does not lead to a reduction in bacterial load compared with the control.

In contrast, the treatments with 0.15 and 0.3% gemifloxacin in solution led to a statistically significant reduction in bacterial load compared to both the control and the treatment with 0.3% aqueous norfloxacin solution, after one day and even more markedly after three days' treatment.

The graph relating to the second experiment (Fig. 9) confirms that all the compared compositions (0.3% gemifloxacin in aqueous solution, 0.3% gemifloxacin in emulsion and 0.3% ofloxacin in aqueous solution) statistically significantly reduce, in the experimental model used, the bacterial load compared to the control; the reduction in bacterial load effected by the gemifloxacin-containing compositions is also statistically significant relative to the reduction effected by ofloxacin.

### 6.2.2. Accumulation of antibiotic in the cornea

Spectrofluorimetric quantitative analysis of the corneas treated with 0.15% and 0.3% gemifloxacin solutions and 0.3% norfloxacin solution (first experiment, Fig 7) indicates that, in the model used, after both one and three days' treatment, gemifloxacin reaches far higher concentrations in this tissue than the MIC₅₀ for *St. aureus* (range MIC₅₀-MIC₉₀ = 0.032-0.064 µg/ml), whereas, after both one and three days' treatment, norfloxacin reaches lower corneal concentrations than its MIC₅₀-MIC₉₀ range for *St. aureus* (0.5-2 µg/ml) (Nai-Xun Chin et al., Antimicrobial Agents and Chemotherapy, 1988, Vol. 32 (5), 656-662).

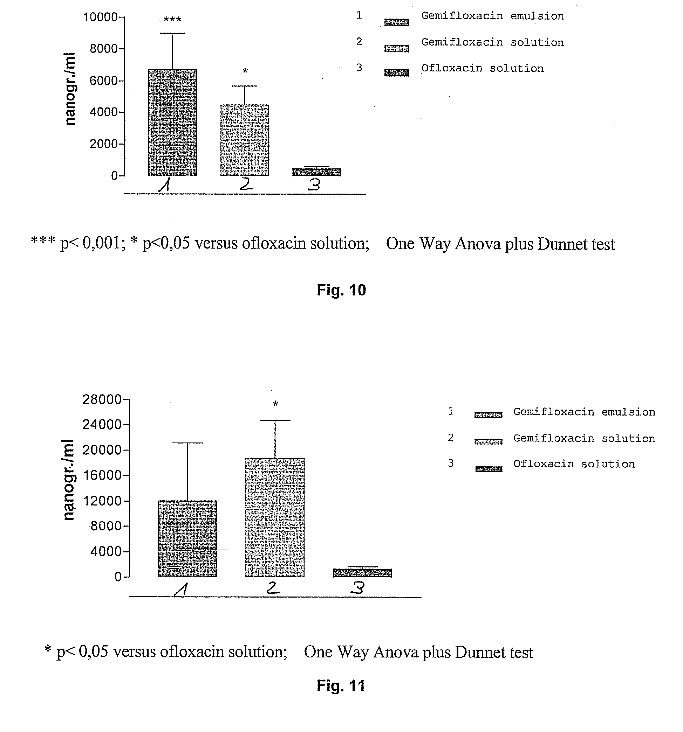

Analysis of the corneas treated with 0.3% gemifloxacin solution and emulsion and with 0.3% ofloxacin solution confirms that in the experimental model used, after one day of treatment, the gemifloxacin reaches, for both compositions, far higher concentrations in this tissue than the MIC_{50/90} (0.032-0.064 µg/ml, established in the second experiment against *St. aureus,* Fig 10). In the same experiment, treatment with 0.3% aqueous ofloxacin solution results in corneal concentrations being near to its MIC₅₀ against *St. aureus* (Fig 10 and Table 1, MIC_{50/90} range = 0.5-1.5).

### 6.2.3. Accumulation of antibiotic in the aqueous humour

Analysis of aqueous humour samples of the 0.15 and 0.3% gemifloxacin solutions and 0.3% norfloxacin solution (first experiment, Fig 8), shows a very substantial passage of gemifloxacin into this region for both gemifloxacin compositions after one day's treatment (10-12 µg/ml), being statistically significant relative to norfloxacin concentrations in the same tissue. Although after three days of treatment gemifloxacin concentrations in the aqueous humour stabilize to between 3-4 µg/ml, said concentrations are once again higher that those determined for norfloxacin in the same tissue and higher than the MIC₅₀-MIC₉₀ range of gemifloxacin for *St. aureus* (0.032-0.064 µg/ml tissue).

The quantitative analysis of gemifloxacin and ofloxacin in the aqueous humour samples relating of the second experiment (Fig. 11) shows that, after one day of treatment, both 0.3% gemifloxacin in solution and 0.3% gemifloxacin in emulsion result in active principle concentrations of between 12 and 18 µg/ml of tissue, values which are far above the MIC_{5o/90} range of gemifloxacin for *St.aureus.*

In the same experiment, the ofloxacin solution reaches concentrations in the aqueous humour equal to about 1.5 µg/ml, near to its MIC_{50/90} range for *St. aureus*

### (0.5-1.15 µg/ml, Table 1)

### Conclusions

The experimental data from the *in vitro* and *in vivo* effectiveness studies (in multi-administration) show that gemifloxacin, in the compositions and at the concentrations used, reaches, in the intrastromal keratitis experimental model, suitable corneal and aqueous humour concentrations for providing the required pharmacological effect with low risk of bacterial resistance arising (concentrations determined as being far higher than MIC_{50/90} values).

Taking into consideration the normal dosage regimen for ophthalmic fluoroquinolones (at least 5 administrations in the first day, followed by 3-5 days of treatment with 3-4 administrations per day), the IQ value can be extrapolated on the basis of the maximum concentration reached in each specific tissue after multi-administration. The IQ value for gemifloxacin, with the multi-administration dosage regimen, proves to be always greater than 1 in all the ocular regions.

The experimental data therefore shows that gemifloxacin is effective in the treatment of ocular infections, presenting lower MIC₉₀ values than third and fourth generation fluoroquinolones against the most common ocular pathogens, higher concentrations in ocular tissues than third generation fluoroquinolones commonly used in the ophthalmic field and, therefore, optimal IQs for ascertaining their pharmacological effect (which, for fluoroquinolones, is concentration dependent), with low risk of bacterial resistance arising.

## Claims

1. Use of gemifloxacin and derivatives thereof for preparing ophthalmic compositions suitable for preventing and treating ocular infections.

2. Use of gemifloxacin and derivatives thereof according to claim 1, wherein the ocular infections are caused by the most common bacterial agents such as: *S. aureus, S. epidermidis,* Coagulase Negative Staphylococci (CNS), Streptococci, *Ps. aeruginosa, S. marcescens, H. influenzae*.

3. Use of gemifloxacin according to claim 1 in racemic form or in separated enantiomers thereof.

4. Use of gemifloxacin according to claim 1 in compositions suitable for ocular administration.

5. Use of gemifloxacin according to claim 4, wherein the compositions are in the form of solutions, emulsions, ointments, solid inserts, suspensions, gels, oily solutions, solutions obtained by means of cyclodextrins, liposomes, nanoparticles, micelles.

6. Use of gemifloxacin in compositions according to claims 4 and 5, in concentrations equal to 0.01-1.0%, preferably in concentrations equal to 0.05-0.5% and even more preferably in concentrations equal to 0.15-0.3%.

7. Ophthalmic pharmaceutical compositions comprising gemifloxacin for the treatment of ocular infections.

8. Ophthalmic pharmaceutical compositions according to claim 7 for topical ocular application or for subconjunctival or intraocular injection.

9. Ophthalmic pharmaceutical compositions according to claim 7, for the preparation of medicated intraocular lenses (IOLs) or medicated corneal lenses.

10. Ophthalmic pharmaceutical compositions according to claim 7, comprising gemifloxacin in amounts comprised between 0.01 and 1.0%, preferably between 0.05 and 0.5%, and even more preferably between 0.15 and 0.3%, and a pharmaceutically acceptable vehicle.

11. Ophthalmic pharmaceutical compositions according to claim 7, comprising gemifloxacin in combination with corticosteroids.

12. Ophthalmic pharmaceutical compositions according to claim 7, comprising gemifloxacin in combination with non-steroidal anti-inflammatories.

13. Ophthalmic pharmaceutical compositions according to claim 7, comprising gemifloxacin in combination with beta-lactam antibiotics.

14. Ophthalmic pharmaceutical compositions according to claim 13, wherein the beta-lactam antibiotic is a cephalosporin.

15. Ophthalmic pharmaceutical compositions according to claim 13, wherein the beta-lactam antibiotic is a penicillin.

16. Ophthalmic pharmaceutical compositions according to claim 7, comprising gemifloxacin in combination with macrolides.

17. Ophthalmic pharmaceutical compositions according to claim 16, wherein the macrolide is azithromycin.

18. Ophthalmic pharmaceutical compositions according to claim 16, wherein the macrolide is erythromycin.

19. Ophthalmic pharmaceutical compositions according to claim 7, comprising gemifloxacin in combination with aminoglycosides.

20. Ophthalmic pharmaceutical compositions according to claim 19, wherein the aminoglycoside is netilmycin.

21. Ophthalmic pharmaceutical compositions according to claim 7, wherein the pharmaceutical composition is in the form of a solution, emulsion, ointment, medicated solid inserts, suspensions, gels, solutions obtained by using cyclodextrins, liposomes, nanoparticles or micelles, suitable for topical optical application or subconjunctival or intraocular injection.
